# EUROPÄISCHE PATENTSCHRIFT

(11) **EP 4 205 715 B1**
(45) Veröffentlichungstag und Bekanntmachung des Hinweises auf die Patenterteilung: **28.01.2026**
(21) Anmeldenummer: 22210714.6
(22) Anmeldetag: 01.12.2022
(51) Int. Cl.: A61F 13/15

(54) **VERFAHREN ZUM DOSIEREN UND APPLIZIEREN EINER KLEINEN MENGE EINES PARTIKULÄREN MATERIALS**
METHOD FOR DOSING AND APPLYING A SMALL AMOUNT OF A PARTICULATE MATERIAL
PROCÉDÉ DE DOSAGE ET D'APPLICATION D'UNE PETITE QUANTITÉ DE MATÉRIAU PARTICULAIRE

(30) Priorität: 29.12.2021 DE 102021006409
(43) Veröffentlichungstag der Anmeldung: 05.07.2023
(73) Patentinhaber: Paul Hartmann AG, 89522 Heidenheim (DE)
(72) Erfinder: JÜRGENS, Ralf, 89522 Heidenheim (DE); KNECHT, Theresia, 73433 Aalen (DE); FRANK, Bernd, 89555 Steinheim (DE); ACIKEL, Ümit, 73447 Oberkochen (DE); STEIGERWALD, Jörg, 89407 Dillingen (DE)
(74) Vertreter: DREISS Patentanwälte PartG mbB

(56) Entgegenhaltungen:
- EP-A2- 0 347 544
- US-A- 5 072 687

## Beschreibung

Die Erfindung betrifft ein Verfahren zum Dosieren und Applizieren einer kleinen Menge eines partikulären Materials in einer schnelllaufenden Herstellungsmaschine für absorbierende Hygieneartikel, wobei in der Herstellungsmaschine aufeinanderfolgend Komponenten der herzustellenden Hygieneartikel als Zielstruktur für das zu applizierende partikuläre Material zugeführt und gefördert werden, wobei eine Dosierwalze mit in ihrer Außenumfangsfläche vorgesehenen Kavitäten zur Aufnahme des partikulären Materials eingesetzt wird, wobei die aufeinanderfolgend zugeführten Komponenten an und vorzugsweise unterhalb der Dosierwalze und vorzugsweise in einem Abstand zur Dosierwalze vorbeigeführt werden.

Verfahren und Vorrichtungen zum Dosieren und Applizieren von superabsorbierenden Partikeln unter Verwendung von kontinuierlich angetriebenen Dosierwalzen sind beispielsweise bekannt aus WO 13/0084311 A1, EP 1 655 007 A1, EP 2 583 648 A1. Hierbei werden beträchtliche Mengen superabsorbierenden Granulats zur Bildung eines SAP Laminats zwischen Flachmaterialien eingebracht. Bei einem anderen Verfahren gemäß EP 2 777 664 B1 wird ein superabsorbierendes Partikelmaterial in einen Behälter gegeben, und eine Austrittsöffnung aus dem Behälter wird mittels eines hin und her schwenkbaren keilförmigen Elements abwechselnd geöffnet und geschlossen, so dass eine jeweils möglichst gleiche und vorbestimmte Menge an Material herausrieseln und auf ein darunter laufendes Band bzw. eine dort geförderte Zielstruktur gelangen kann.

Wenn superabsorbierendes partikuläres Material in Komponenten von Hygieneartikeln appliziert wird, so handelt es sich hierbei typischerweise um eher erhebliche Mengen von wenigstens 5,0 g, insbesondere wenigstens 8,0 g, insbesondere wenigstens 10,0 g partikulären Materials. Demgegenüber befasst sich die vorliegende Erfindung mit einem Verfahren zum Dosieren und Applizieren einer kleineren Menge von beispielsweise höchstens 2,0 g oder höchstens 1,0 g partikulären Materials. Es kann sich hierbei beispielsweise um ein pH-Regulationsmittel, insbesondere eine Säure oder deren Salze oder eine Mischung davon, insbesondere Mono-Natrium-Citrat oder Di-Natrium-Citrat, handeln, welches einem eher eng begrenzten Bereich einer Absorptionskörperkomponente zugeführt und appliziert werden soll. Ein Verfahren mit den Merkmalen des Oberbegriffs des Anspruchs 1 ist bekannt aus EP 0 347 544 A2.

Typischerweise vorbekannte Vorrichtungen und Verfahren zum Dosieren und Applizieren von je Artikel größeren Mengen superabsorbierenden Materials sind nicht geeignet, um sehr geringe Mengen eines möglicherweise auch nicht optimal rieselfähigen, also kleinkörnigen oder pulverförmigen partikulären Materials in einer vorgegebenen geringen Menge zielgenau zu applizieren. Bei den bekannten Vorrichtungen und Verfahren wird partikuläres Material entweder mehr oder weniger kontinuierlich ausgegeben, oder es können nur breite Gaußverteilungen an Partikelmengen appliziert werden, was mit einer nicht konstanten Applikationsmenge und einer nicht zufriedenstellenden flächenmäßigen Verteilung oder Applikation des Materials auf der Zielstruktur einhergeht.

Wenn Dosierwalzen zum Einsatz gelangen, dann werden sie mit gleichbleibender Geschwindigkeit betrieben und zumeist gegenüber der zu beschickenden Komponente abgerollt. Ihre Kavitäten sind entweder gleichmäßig über den Außenumfang der Walze verteilt angeordnet, oder auch in Gruppen, um partikuläres Material an in einer Maschinenrichtung der Herstellungsmaschine beabstandete Komponenten zu applizieren. Dies bedeutet aber, dass je nach Erstreckung des Zielbereichs in der Maschinenrichtung und je nach Beabstandung der Komponenten in Maschinenrichtung voneinander jeweils individuell angepasste Dosierwalzen angefertigt, vorgehalten und verwendet werden müssen.

Hiervon ausgehend liegt der vorliegenden Erfindung die Aufgabe zugrunde, ein Verfahren und eine Vorrichtung zum genaueren Dosieren und zielgenauen Applizieren einer kleinen Menge partikulären Materials in einer schnelllaufenden Herstellungsmaschine für absorbierende Hygieneartikel anzugeben.

Diese Aufgabe wird gelöst durch ein Verfahren der eingangs genannten Art, das dadurch gekennzeichnet ist, dass die Dosierwalze getaktet angesteuert wird, also entsprechend der Bewegung der vorbeigeführten Komponenten beschleunigt und in jedem Takt bis zum Stillstand verzögert wird, so dass mit jedem Takt eine Anzahl von Kavitäten in eine Abgabeposition zu der gerade vorbeigeführten Komponente gebracht und darin enthaltenes partikuläres Material an die Komponente appliziert wird.

Es wurde erfindungsgemäß festgestellt, dass sich das Problem des Dosierens und zielgenauen Applizierens kleiner Mengen partikulären Materials durch einen getakteten, also intermittierenden Betrieb der Dosierwalze zufriedenstellend lösen lässt und dass damit auch ein wirtschaftlicher Betrieb der Herstellungsmaschine für Hygieneartikel möglich ist. Durch einen getakteten Antrieb kann durch Einsatz ein und derselben Dosierwalze je nach Drehwinkel eine mehr oder weniger große Anzahl von mit partikulärem Material befüllten Kavitäten in die Abgabeposition gebracht werden. Hierbei wird unter einer Abgabeposition der Kavitäten der Dosierwalze verstanden, dass diese Kavitäten gegenüber der Zielstruktur exponiert sind, also nicht mehr durch Vorrichtungskomponenten, insbesondere eine ihre Entleerung verhindernde Rakeleinrichtung abgedeckt sind, so dass das darin enthaltene partikuläre Material schwerkraftunterstützt und/oder durch Zentripetalbeschleunigungen der Dosierwalze unterstützt und/oder durch die Beschleunigung und Verzögerung der Dosierwalze unterstützt ausgegeben und vorzugsweise direkt oder indirekt auf die Zielstruktur gelangt oder geleitet wird.

Durch einen solchen getakteten und damit intermittierenden Antrieb der Dosierwalze lässt sich eine vorbestimmte Menge an partikulärem Material im richtigen Moment und zielgenau auf eine Zielstruktur applizieren. Es erweist sich hierbei als vorteilhaft, dass die Dosierwalze in jedem Takt bis zum Stillstand verzögert wird. Dies ist mit hohen Beschleunigungen und Verzögerungen verbunden, die sich auf eine vollständige Entleerung der Kavitäten vorteilhaft auswirken bzw. dies unterstützen. Es erweist sich als vorteilhaft, dass unter Anwendung des erfindungsgemäßen Verfahrens auch partikuläres Material mit nicht ganz optimaler Rieselfähigkeit vollständig aus den Kavitäten ausgegeben und auf die Zielkomponente appliziert werden kann. Während typische superabsorbierende partikuläre Materialien eine Partikelgröße von überwiegend größer 300 µm aufweisen und als gut rieselfähig angesehen werden können, also beispielsweise wenigstens 80 Gewichtsprozent einer Partikelfraktion werden bei einer Siebrückstandsuntersuchung durch ein Sieb mit Öffnungen von 300 µm zurückgehalten, so ist dies bei demgegenüber feinerem, eher in Richtung pulverförmig gehendem partikulärem Material mitunter problematisch. Dennoch lassen sich durch den getakteten intermittierenden Antrieb der Dosierwalze auch partikuläre Materialien mit einer Partikelgröße von überwiegend kleiner 300 µm (wenigstens 80 Gewichtsprozent einer Partikelfraktion passieren bei einer Siebrückstandsuntersuchung ein Sieb mit Öffnungen von 300 µm) mit hoher Dosiergenauigkeit applizieren, indem das Material aus den Kavitäten für praktische Anwendungen rückstandslos ausgegeben wird.

Weiter kann ein und dieselbe Dosierwalze für die Herstellung von Hygieneartikeln mit unterschiedlicher Menge des zu applizierenden partikulären Materials je Hygieneartikel verwendet werden. Je nach gewünschter Dosis oder Menge des zu applizierenden partikulären Materials wird ein Drehwinkel der Dosierwalze je Takt gewählt. Je größer der Drehwinkel je Takt desto mehr mit partikulärem Material gefüllte Kavitäten kommen in die Abgabeposition und werden dabei entleert. Hierdurch kann die Dosiermenge je Artikel gewissermaßen stufenlos gewählt werden. Da die Kavitäten auch sehr klein gewählt werden können, können auch geringe Mengen an partikulärem Material genau dosiert und appliziert werden.

Es erweist sich bei der Ansteuerung der Dosierwalze als vorteilhaft, wenn der Drehwinkel zwischen 5° und 30°, insbesondere zwischen 10° und 25° gewählt wird.

Die vorliegende Erfindung erweist sich als besonders vorteilhaft, wenn eine Maschinengeschwindigkeit von wenigstens 100 Komponenten der herzustellenden Hygieneartikeln pro Minute, insbesondere von wenigstens 200 Komponenten der herzustellenden Hygieneartikel pro Minute, insbesondere von wenigstens 300 Komponenten der herzustellenden Hygieneartikel pro Minute, insbesondere von wenigstens 400 Komponenten der herzustellenden Hygieneartikel pro Minute, insbesondere von etwa 500 Komponenten der herzustellenden Hygieneartikel pro Minute gefahren wird.

Insbesondere erweist es sich als vorteilhaft, wenn eine Maschinengeschwindigkeit bei der Zuführung der Komponenten von wenigstens 1 m/s, insbesondere von wenigstens 2 m/s, insbesondere von wenigstens 3 m/s, insbesondere von wenigstens 4 m/s, und weiter insbesondere von wenigstens 5 m/s gefahren wird. Dies führt dazu, dass sich eine Zielfläche der Komponente lediglich in der Größenordnung von 20-100 ms, insbesondere 40 - 60 ms und typischerweise nur 50 ms, lang in Projektion vertikal unterhalb der Dosierwalze befindet. Dementsprechend muss die getaktete Ansteuerung der Dosierwalze ausgeführt werden.

Es erweist sich weiter im Hinblick auf eine zielgenaue Positionierung und angesichts einer tangentialen Komponente des partikulären Materials beim Abgeben oder Herausschleudern aus den Kavitäten in der Abgabeposition als vorteilhaft, wenn der Abgabeposition der Kavitäten eine Leitanordnung nachgeordnet ist, mittels derer aus den Kavitäten ausgegebenes partikuläres Material in Richtung auf die Zielstruktur oder einen Zielbereich der Zielstruktur geleitet wird.

Hierbei erweist es sich als vorteilhaft, wenn die Leitanordnung eine Prall- oder Leitwand aufweist, welche zu einer horizontalen Ebene einen Winkel zwischen 90° und 20°, insbesondere zwischen 90° und 25°, insbesondere zwischen 80° und 25°, insbesondere zwischen 60° und 25° und weiter insbesondere zwischen 50° und 25° einschließt.

Es ist auch denkbar und vorteilhaft, wenn die Prall- oder Leitwand je nach Positionierung zur Außenumfangsfläche der Dosierwalze in der Abgabeposition unterschiedliche Winkel zur horizontalen Ebene einschließt.

Die Kavitäten bei der Dosierwalze haben die Aufgabe, partikuläres Material dosiergenau aufzunehmen und vollständig abzugeben, wenn sie in die Abgabeposition gebracht und dort insbesondere bis zum Stillstand verzögert werden. Sie sollten also mit wohldefiniertem Aufnahmevolumen ausgebildet sein. Ferner soll eine gute Entleerbarkeit gewährleistet werden. Insbesondere im Hinblick auf diesen Aspekt erweist es sich als vorteilhaft, wenn sich nach innen verjüngende Kavitäten, insbesondere verrundet ausgebildete sich nach innen verjüngende Kavitäten, insbesondere kalottenförmig ausgebildete Kavitäten bei der Dosierwalze vorgesehen werden.

Weiter erweist es sich als vorteilhaft, wenn ein Aufnahmevolumen einer Kavität wenigstens 6 mm³, insbesondere wenigstens 8 mm³, insbesondere wenigstens 10 mm³ und weiter insbesondere höchstens 20 mm³, insbesondere höchstens 15 mm³, insbesondere höchstens 13 mm³ beträgt.

Weiter erweist es sich als vorteilhaft, wenn eine Tiefe einer Kavität wenigstens 1,0 mm, insbesondere wenigstens 1,5 mm, insbesondere höchstens 4,0 mm, insbesondere höchstens 3,0 mm beträgt.

Weiter erweist es sich als vorteilhaft, wenn eine größte Abmessung einer lichten Querschnittsfläche einer Kavität in der Außenumfangsfläche der Dosierwalze wenigstens 2,5 mm, insbesondere wenigstens 3,0 mm, insbesondere höchstens 5,0 mm, insbesondere höchstens 4,5 mm, insbesondere höchstens 4,0 mm beträgt.

Um eine vollständige Entleerung der Kavitäten in der Abgabeposition zu unterstützen, erweist es sich als vorteilhaft, wenn die Kavitäten in einem Übergang zu der Außenumfangsfläche der Dosierwalze von einer Wandung mit einem Öffnungswinkel von höchstens 60°, insbesondere von höchstens 50°, insbesondere von höchstens 45° und von wenigstens 25°, insbesondere von wenigstens 30°, insbesondere von wenigstens 35° begrenzt werden.

Im Hinblick auf die Erzielung einer hohen Dosiergenauigkeit erweist es sich als vorteilhaft, dass die Kavitäten derart ausgebildet und in der Außenumfangsfläche der Dosierwalze angeordnet sind, dass sie in der Abgabeposition vollständig exponiert sind, also nicht durch eine Rakeleinrichtung oder deren Rakelschneide teilweise überdeckt und teilweise freigegeben sind. Dies kann beispielsweise sehr einfach erreicht werden, wenn die Kavitäten in der Außenumfangsfläche der Dosierwalze in parallel zu einer Antriebsachse der Dosierwalze verlaufenden Reihen angeordnet sind.

Die Kavitäten müssen aber nicht streng entsprechend einem quadratischen Muster angeordnet sein. Es kann sich auch als vorteilhaft erweisen, wenn die Kavitäten in der Außenumfangsfläche von Reihe zu Reihe in Richtung der Antriebsachse versetzt zueinander angeordnet sind. Auf diese Weise kann eine höhere Dichte an Kavitäten auf der Außenumfangsfläche realisiert werden.

Es kann sich auch als denkbar und vorteilhaft erweisen, wenn die Kavitäten in der Außenumfangsfläche der Dosierwalze in Gruppen angeordnet sind, die in Umfangsrichtung voneinander beabstandet sind. So lässt sich realisieren, dass in Umfangsrichtung zwischen diesen Gruppen ein kavitätenfreier Steg ausgebildet ist. Auf diesem Steg kann dann eine Rakelschneide zwischen zwei aufeinanderfolgenden Gruppen von Kavitäten positioniert sein und anliegen, wobei sich die eine Gruppe in der Abgabeposition und die andere Gruppe noch vor der Abgabeposition und durch die Rakelschneide abgedeckt befindet.

Es kann sich als vorteilhaft erweisen, wenn ein Außendurchmesser der Dosierwalze wenigstens 60 mm, insbesondere wenigstens 70 mm, insbesondere wenigstens 80 mm, und insbesondere höchstens 350 mm, insbesondere höchstens 320 mm, insbesondere höchstens 300 mm, insbesondere höchstens 250 mm, insbesondere höchstens 200 mm, insbesondere höchstens 150 mm aufweist.

Wie eingangs schon erwähnt, bezieht sich die vorliegende Erfindung insbesondere auf ein Verfahren, bei dem je Komponente und Takt eine geringe Menge partikulären Materials von höchstens 2,0 g, insbesondere von höchstens 1,5 g, insbesondere von höchstens 1,2 g, insbesondere von höchstens 1,0 g, insbesondere von höchstens 0,8 g appliziert wird.

Gegenstand der Erfindung ist weiter eine Vorrichtung zum Dosieren und Applizieren einer kleinen Menge eines partikulären Materials in einer schnelllaufenden Herstellungsmaschine für absorbierende Hygieneartikel, wobei in der Herstellungsmaschine aufeinanderfolgend Komponenten der herzustellenden Hygieneartikel als Zielstruktur für das zu applizierende partikuläre Material zugeführt und gefördert werden,
mit einer Dosierwalze mit in ihrer Außenumfangsfläche vorgesehenen Kavitäten zur Aufnahme des partikulären Materials, und
mit einer Rakeleinrichtung mit einer gegen die Außenumfangsfläche der Dosierwalze anliegenden Rakelschneide,
wobei die aufeinanderfolgend zugeführten Komponenten an und vorzugsweise unterhalb der Dosierwalze und vorzugsweise in einem Abstand zur Dosierwalze vorbeigeführt werden, und mit einer Steuer- und Antriebsvorrichtung für die Dosierwalze, dadurch gekennzeichnet,
dass die Steuer- und Antriebsvorrichtung derart ausgebildet ist, dass die Dosierwalze getaktet ansteuerbar ist, also entsprechend der Bewegung der vorbeigeführten Komponenten beschleunigt und in jedem Takt bis zum Stillstand verzögert wird, so dass mit jedem Takt eine Anzahl von Kavitäten in eine Abgabeposition zu der gerade vorbeigeführten Komponente gebracht und darin enthaltenes partikuläres Material an die Komponente appliziert wird.

Die Vorrichtung kann vorteilhafterweise so ausgebildet werden, dass je nach Menge an zu applizierendem partikulären Material ein Drehwinkel der Dosierwalze je Takt, insbesondere zwischen 5° und 30°, wählbar ist.

Ein vertikaler Abstand der an der Dosierwalze vorbeigeführten Komponenten und der Dosierwalze kann vorteilhafterweise 1,0 - 15,0 cm, insbesondere 1,0 - 10,0, insbesondere 1,0 - 7,0 cm, insbesondere 1,0 - 5,0 cm, insbesondere 1,0 - 4,0 cm, insbesondere 1,0 - 3,0 cm, insbesondere 2,0 - 3,0 cm betragen.

Die Vorrichtung kann vorteilhafterweise so ausgebildet werden, dass der Abgabeposition der Kavitäten eine Leitanordnung nachgeordnet ist, mittels derer aus den Kavitäten ausgegebenes partikuläres Material in Richtung auf die Zielstruktur oder einen Zielbereich der Zielstruktur geleitet wird.

Die Vorrichtung kann vorteilhafterweise so ausgebildet werden, dass die Leitanordnung eine Prall- oder Leitwand aufweist, welche zu einer horizontalen Ebene einen Winkel zwischen 90° und 20°, insbesondere zwischen 90° und 25°, insbesondere zwischen 80° und 25°, insbesondere zwischen 60° und 25° und weiter insbesondere zwischen 50° und 25° einschließt.

Die Vorrichtung kann vorteilhafterweise so ausgebildet werden, dass bei der Dosierwalze sich verjüngende Kavitäten, insbesondere verrundet ausgebildete sich verjüngende Kavitäten, insbesondere kalottenförmig ausgebildete Kavitäten ausgebildet sind.

Die Vorrichtung kann vorteilhafterweise so ausgebildet werden, dass ein Aufnahmevolumen einer Kavität wenigstens 6 mm³, insbesondere wenigstens 8 mm³, insbesondere wenigstens 10 mm³ und weiter insbesondere höchstens 20 mm³, insbesondere höchstens 15 mm³, insbesondere höchstens 13 mm³ beträgt.

Die Vorrichtung kann vorteilhafterweise so ausgebildet werden, dass eine Tiefe einer Kavität wenigstens 1,0 mm, insbesondere wenigstens 1,5 mm, insbesondere höchstens 4,0 mm, insbesondere höchstens 3,0 mm beträgt.

Die Vorrichtung kann vorteilhafterweise so ausgebildet werden, dass eine größte Abmessung einer lichten Querschnittsfläche einer Kavität in der Außenumfangsfläche der Dosierwalze wenigstens 2,5 mm, insbesondere wenigstens 3,0 mm, insbesondere höchstens 5,0 mm, insbesondere höchstens 4,5 mm, insbesondere höchstens 4,0 mm beträgt.

Die Vorrichtung kann vorteilhafterweise so ausgebildet werden, dass die Kavitäten in einem Übergang zu der Außenumfangsfläche der Dosierwalze von einer Wandung mit einem Öffnungswinkel von höchstens 60°, insbesondere von höchstens 50°, insbesondere von höchstens 45° und von wenigstens 25°, insbesondere von wenigstens 30°, insbesondere von wenigstens 35° begrenzt werden.

Die Vorrichtung kann vorteilhafterweise so ausgebildet werden, dass die Kavitäten in der Außenumfangsfläche der Dosierwalze in parallel zu einer Antriebsachse der Dosierwalze verlaufenden Reihen angeordnet sind.

Die Vorrichtung kann vorteilhafterweise so ausgebildet werden, dass die Kavitäten in der Außenumfangsfläche von Reihe zu Reihe in Richtung der Antriebsachse versetzt zueinander angeordnet sind.

Die Vorrichtung kann vorteilhafterweise so ausgebildet werden, dass die Kavitäten in der Außenumfangsfläche der Dosierwalze in Gruppen angeordnet sind, die in Umfangsrichtung voneinander beabstandet sind, so dass in Umfangsrichtung zwischen diesen Gruppen ein kavitätenfreier Stegbereich ausgebildet ist.

Die Vorrichtung kann vorteilhafterweise so ausgebildet werden, dass ein Außendurchmesser der Dosierwalze wenigstens 60 mm, insbesondere wenigstens 70 mm, insbesondere wenigstens 80 mm, und insbesondere höchstens 350 mm, insbesondere höchstens 320 mm, insbesondere höchstens 300 mm, insbesondere höchstens 250 mm, insbesondere höchstens 200 mm, insbesondere höchstens 150 mm aufweist.

Die Vorrichtung kann vorteilhafterweise so ausgebildet werden, dass je Komponente und Takt eine geringe Menge partikulären Materials von höchstens 2,0 g, insbesondere von höchstens 1,5 g, insbesondere von höchstens 1,2 g, insbesondere von höchstens 1,0 g, insbesondere von höchstens 0,8 g appliziert wird.

Gegenstand der Erfindung ist weiter eine Herstellungsmaschine für absorbierende Hygieneartikel mit einer Vorrichtung zum Dosieren und Applizieren einer kleinen Menge eines partikulären Materials, wie sie vorausgehend beschrieben und in den beigefügten Patentansprüchen beansprucht wurde.

Weitere Merkmale, Einzelheiten und Vorteile der Erfindung ergeben sich aus den beigefügten Patentansprüchen und aus der beigefügten zeichnerischen Darstellung und nachfolgenden Beschreibung der Erfindung. In der Zeichnung zeigt:
Figur 1 eine Schnittansicht durch eine erfindungsgemäße Vorrichtung zum Dosieren und Applizieren einer kleinen Menge eines partikulären Materials, mit unterhalb der Vorrichtung vorbeigeführten Komponenten eines herzustellenden Hygieneartikels und nachgeordneter Topsheetzuführung;
Figur 2 eine Ansicht einer Dosierwalzeneinheit der Vorrichtung nach Figur 1 gesehen in Richtung des Pfeils II in Figur 1;
Figur 3 eine perspektivische Ansicht der Dosierwalzeneinheit nach Figur 2;
Figur 4 eine weitere perspektivische Ansicht der Dosierwalzeneinheit nach Figur 2;
Figuren 5 und 6 verschiedene Muster der Anordnung von Kavitäten auf der Dosierwalze;
Figuren 7 a-d verschiedene Ansichten der Dosierwalze mit Antriebswelle und Einzelheiten der Kavitäten;
Figuren 8a-h weitere beispielhafte Anordnungen der Kavitäten durch eine Aufsicht in radialer Richtung auf eine jeweilige Dosierwalze.

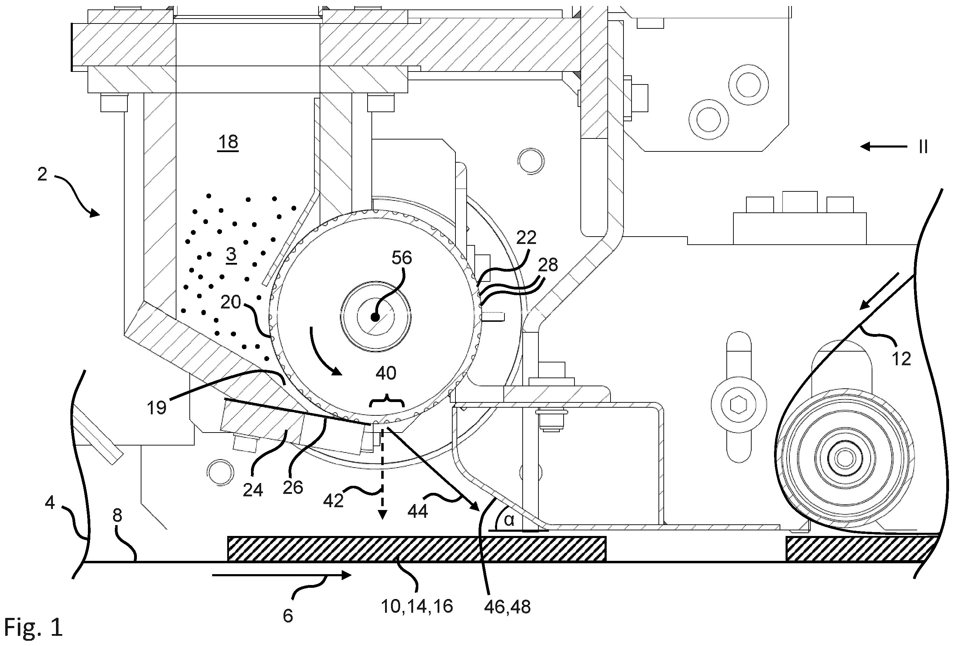

Figur 1 zeigt eine insgesamt mit dem Bezugszeichen 2 bezeichnete Vorrichtung zum Dosieren und Applizieren einer kleinen Menge eines partikulären Materials 3, beispielsweise in Form eines pH-Regulationsmittels, in einer nur teilweise angedeuteten Herstellungsmaschine 4 für absorbierende Hygieneartikel. Von dieser Herstellungsmaschine 4 ist nur ein Teil gezeigt, in dem eine in einer Maschinenrichtung 6 endlos zugeführte Trägermaterialbahn 8, etwa zur Bildung eines Backsheets des Hygieneartikels, mit darauf in der Maschinenrichtung 6 zueinander beabstandet angeordneten Absorptionskörpern 10 zugeführt und beispielhaft und bevorzugt unterhalb der Vorrichtung 2 vorbeigeführt werden. Der Vorrichtung 2 nachgeordnet wird dann eine weitere endlose Materialbahn 12, etwa in Form eines flüssigkeitsdurchlässigen Topsheets des Hygieneartikels, zugeführt, so dass ein jeweiliger Absorptionskörper 10 sandwichartig zwischen der Trägermaterialbahn 8 und der Materialbahn 12 aufgenommen ist.

Ein jeweiliger in der Maschinenrichtung 6 zugeführter Absorptionskörper 10 bildet eine Komponente 14 eines jeweiligen herzustellenden Hygieneartikels, in welche oder an welche als Zielstruktur 16 eine bestimmte vorgegebene Menge des partikulären Materials 3 möglichst zielgenau appliziert werden soll. Hierfür umfasst die Vorrichtung 2 einen Vorratsbehälter 18 zur Aufnahme des in Figur 1 nur angedeuteten partikulären Materials 3 und eine Dosierwalze 20, welche in oder bei einer unteren Öffnung 19 des Vorratsbehälters 18 derart angebracht ist, dass sie mit einem Teil ihrer Außenumfangsfläche 22 von dem partikulären Material 3 im Inneren des Vorratsbehälters 18 beaufschlagt wird und die Öffnung 19 des Vorratsbehälters 18 im Übrigen und zusammen mit einer Rakeleinrichtung 24 bzw. deren Rakelschneide 26 im wesentlichen partikeldicht verschließt.

Die Dosierwalze 20 weist wie erwähnt eine Außenumfangsfläche 22 auf, in der eine Vielzahl von Kavitäten 28 zur Aufnahme des partikulären Materials 3 ausgebildet sind.

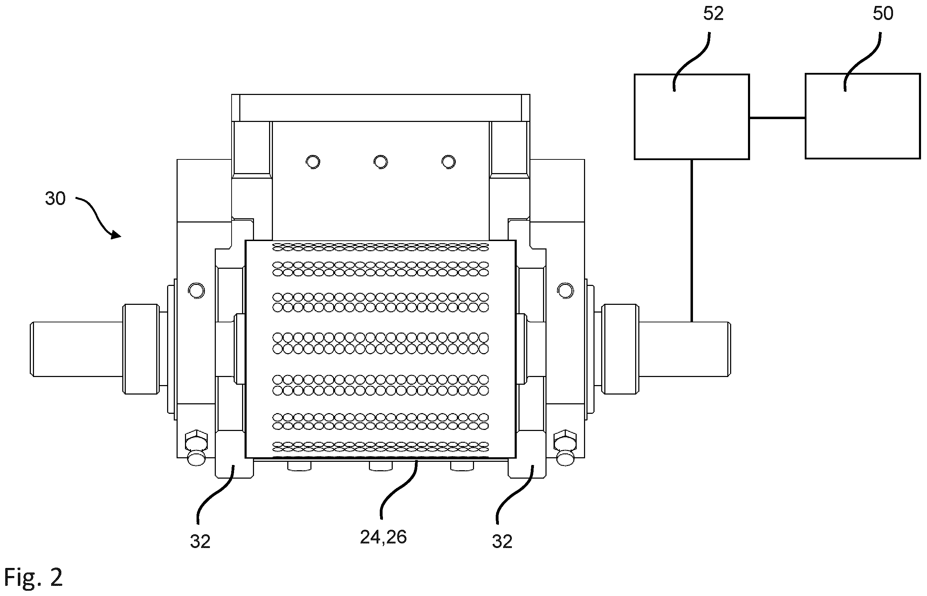

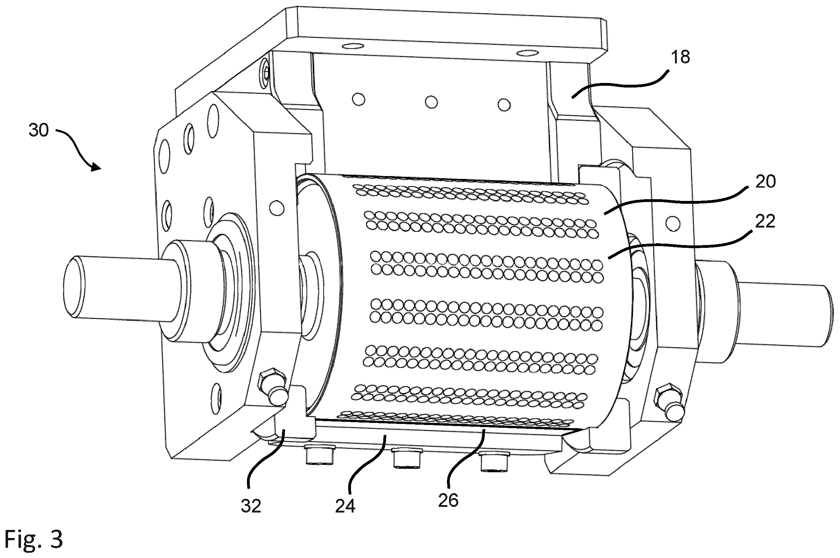

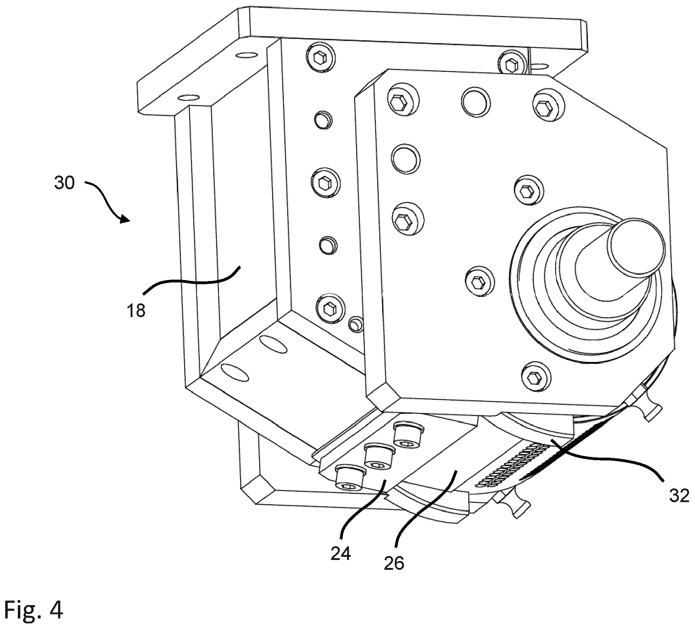

Die Figuren 2 bis 4 zeigen in weiteren Darstellungen die Vorrichtung 2 zur besseren Darstellung unter Weglassung von weiteren Komponenten der Figur 1. Man erkennt, dass der Vorratsbehälter 18 zusammen mit der darin drehbar gelagerten Dosierwalze 20 eine modular anmutende Dosiereinheit 30 bildet, die als Ganzes in der schnelllaufenden Herstellungsmaschine 4 anbringbar bzw. in die Herstellungsmaschine 4 integrierbar ist.

Wenn die Dosierwalze 20 mit ihren Kavitäten 28 in Kontakt mit dem partikulären Material 3 im Inneren des Vorratsbehälters 18 gelangt, so füllen sich die Kavitäten 28 mit diesem partikulären Material 3. Ein Austritt dieses Materials 3 nach außerhalb der Dosiereinheit 30 wird durch die bezüglich der Dosierwalze 20 unten vorgesehene Rakeleinrichtung 24 mit Ihrer Rakelschneide 26 und durch ein jeweiliges Dichtelement 32 im jeweiligen Bereich des Übergangs der Außenumfangsfläche 22 zur angrenzenden axialen Stirnseite der Dosierwalze 20 realisiert. Das Dichtelement 32 ist in vorteilhafter Weise als ein etwas nachgiebiges Kunststoffdichtelement ausgebildet und nur aus Figuren 2 bis 4 ersichtlich. Somit ist das Innere der Vorrichtung 2 bzw. ihres Vorratsbehälters 18 gegen die Umgebung und die Herstellungsmaschine 4 wirksam abgedichtet. Erst wenn jeweilige Kavitäten 28 der Dosierwalze 20 von der Rakelschneide 26 freigegeben werden, kann die darin aufgenommene geringe und genau dosierte Menge des partikulären Materials in Richtung auf die Komponente 14 des Hygieneartikels abgegeben werden. Dies kann bei der hier gezeigten Vorbeiführung der Komponenten 14 beispielhaft unterhalb und beispielhaft in einem vertikalen Abstand von 1-15 cm von der Dosierwalze 20 schwerkraftunterstützt erfolgen. Zum anderen entstehen Zentripetalbeschleunigungen infolge der noch zu erläuternden Umdrehung der Dosierwalze 20 und ferner auch bezüglich dieser Umdrehung tangentiale Komponenten bei der Abgabe des partikulären Materials 3 aus den Kavitäten 28, wenn diese in eine der Rakeleinrichtung 24 unmittelbar nachfolgende Abgabeposition 40 gedreht werden. Daher gelangt das partikuläre Material 3 nicht entlang eines gestrichelt angedeuteten Pfeils 42, also exakt in vertikaler Richtung, aus den Kavitäten 28 auf die Komponenten 14, sondern es folgt einem mehr oder weniger schräg hierzu gerichteten Weg, der lediglich rein schematisch durch einen schräg geneigten Pfeil 44 angedeutet ist. Das Material kann und wird wohl einer bogenförmigen nicht dargestellten Bahn folgen. Zur Unterstützung der zielgenauen Applikation ist auch eine Leitanordnung 46 mit einer Prall- oder Leitwand 48 vorgesehen, welche unterschiedliche Neigungswinkel α zur Horizontalen aufweist. Beispielsweise ist der Neigungswinkel α der Prall- oder Leitwand 48 in einem oberen Bereich nahezu 90°, und in einem unteren Bereich beträgt er eher ca. 40°.

Erfindungsgemäß wird die Dosierwalze 20 nicht mit einer konstanten kontinuierlichen Umdrehungsgeschwindigkeit angetrieben, sondern sie wird getaktet angesteuert und hierbei entsprechend der Bewegung der vorbeigeführten Komponenten 14 beschleunigt und erfindungsgemäß bis zum Stillstand verzögert, so dass mit jedem Takt eine Anzahl von Kavitäten 28 in die Abgabeposition 40 in Bezug auf die gerade vorbeigeführte Komponente 14 gebracht und darin enthaltenes partikuläres Material 3 an die Komponente 14 appliziert wird. Es stellte sich heraus, dass hierdurch eine weitaus genauere Dosierung von partikulärem Material und Abgabe an eine jeweilige Komponente erreicht werden kann. Durch eine entsprechend getaktete Ansteuerung mittels einer elektronischen Steuervorrichtung 50 und einer Antriebsvorrichtung 52 (in Figur 2 schematisch angedeutet) lässt sich erreichen, dass eine jeweils vorbestimmte Anzahl von Kavitäten 28 entsprechend einem je Takt gewählten Drehwinkel für die Dosierwalze 20 in die Abgabeposition 40 gelangt. Durch die sich hierbei ergebenden hohen Beschleunigungen und Verzögerungen wird die vollständige Abgabe des partikulären Materials 3 aus jeder in die Abgabeposition 40 gebrachten Kavität 28 erreicht. Dies kann weiter durch eine noch zu beschreibende Formgebung der Kavitäten 28 und durch Ausbildung der die Kavitäten bildenden Oberfläche der Dosierwalze 20 aus poliertem Hartmetall unterstützt werden.

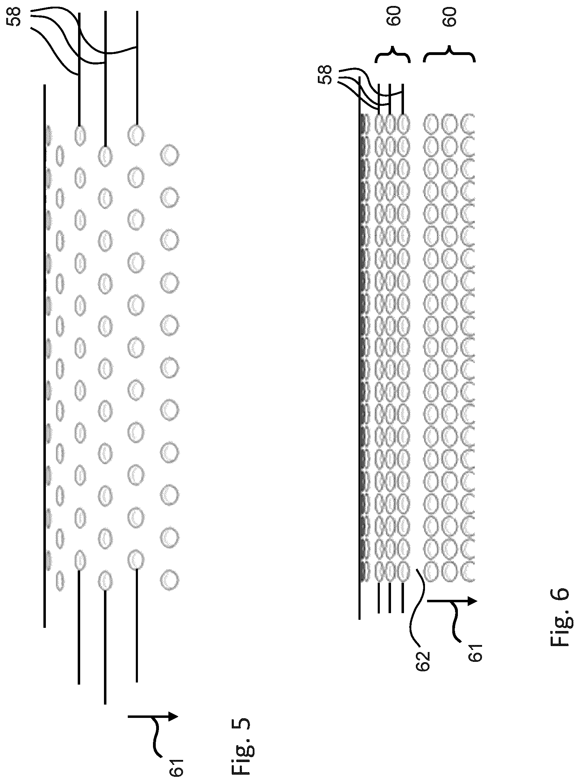

Wie in Figur 1 beispielhaft angedeutet und auch in Figuren 5 und 6 dargestellt, sind die Kavitäten 28 in zu einer geometrischen Antriebsachse 56 parallelen Reihen 58 angeordnet. Zudem erkennt man in Figuren 1 und 6, dass die Kavitäten in Gruppen 60 von jeweils beispielhaft drei Reihen 58 angeordnet sind, die in Umfangsrichtung 61 voneinander beabstandet sind. Figuren 2-4 zeigen beispielhaft Gruppen von jeweils zwei Reihen. Auf diese Weise wird zwischen den Gruppen 60 ein kavitätenfreier Stegbereich 62 gebildet, der sich für eine Anlage der Rakelschneide 26 nach einer inkrementellen Weiterbewegung der Dosierwalze 20 eignet. Es ist aber auch denkbar und vorteilhaft, wenn die Kavitäten 28 auf der Außenumfangsfläche 22 gewissermaßen gleichförmig verteilt angeordnet sind. Dabei erweist es sich als vorteilhaft, wenn die Anordnung derart ist, dass eine Rakelschneide an einer Vielzahl von inkrementellen Drehpositionen der Dosierwalze gegen die Außenumfangsfläche der Dosierwalze anliegen kann, ohne dabei eine Kavität zu überschneiden. Auf diese Weise kann dann eine definierte Anzahl von Kavitäten 28 vollständig in die Abgabeposition 40 gebracht werden.

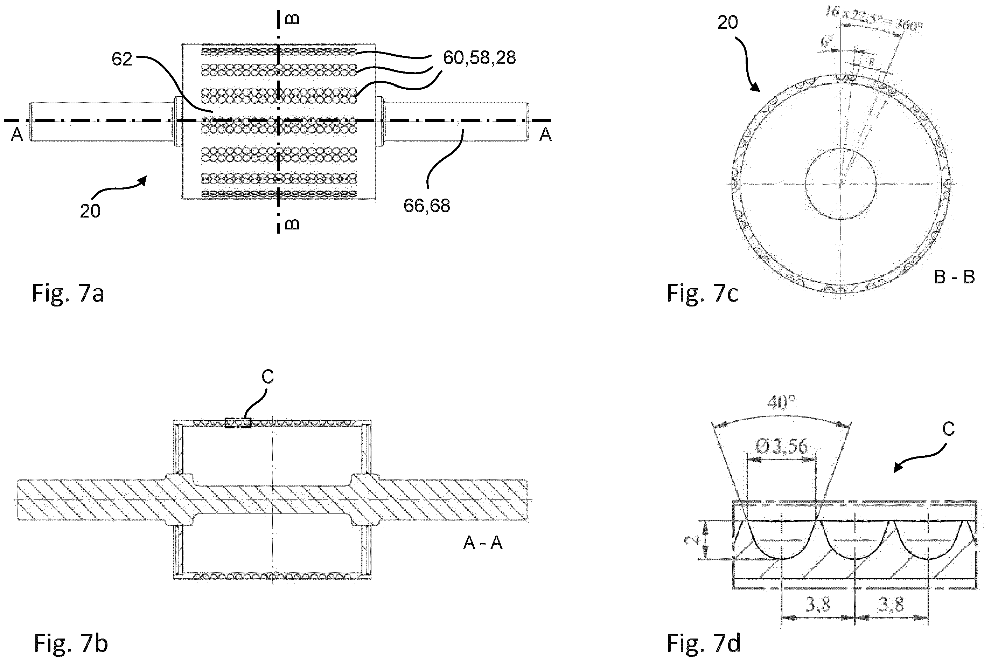

Die Figuren 7a bis 7d zeigen die Dosierwalze 20 mit Lagerabschnitten 66 ihrer Antriebswelle 68. Sie ist vorzugsweise als Hohlwalze ausgebildet. Die Anordnung der Kavitäten 28 ist wiederum beispielhaft in Gruppen 60 von beispielhaft je zwei Reihen 58 von Kavitäten 28 ausgebildet, wobei die Gruppen 60 voneinander durch einen kavitätenfreien Stegbereich 62 beabstandet sind. In Figur 7c (Schnittansicht gemäß der Ebene B-B in Figur 7a) ist hierfür beispielhaft eine Kreissegmentteilung angegeben. Ein Drehwinkel der Dosierwalze 20 beträgt hier beispielhaft 22,5°, was 1/16 einer vollen Umdrehung der Dosierwalze entspricht.

Die Einzelheit C gemäß Figur 7d zeigt, dass die Kavitäten 28 sich in Richtung nach radial innen verjüngend ausgebildet sind. Sie sind in vorteilhafter Weise dabei verrundet ausgebildet und in ihrem radial innen liegenden Scheitelbereich kalottenförmig, insbesondere abschnittsweise kugelkalottenförmig ausgebildet. Man erkennt weiter, dass die Kavitäten 28 in einem Übergang zu der Außenumfangsfläche der Dosierwalze von einer Wandung mit einem hier beispielhaft mit 40° angegebenen Öffnungswinkel begrenzt ist. Die Abmessungsangaben (in Millimeter) der Kavitäten in den Figuren sind aber rein beispielhaft.

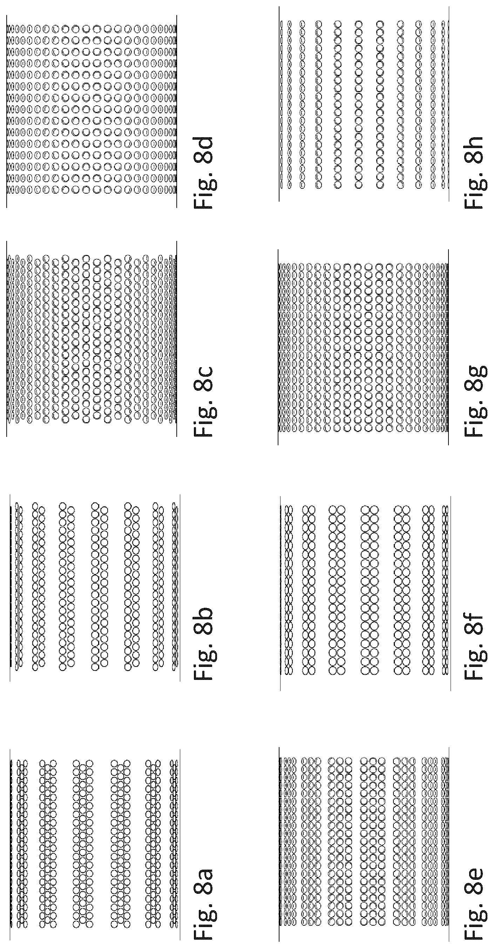

Die Figuren 8a-h zeigen weitere beispielhafte Anordnungen der Kavitäten durch eine Aufsicht in radialer Richtung auf eine jeweilige Dosierwalze. Je dichter die Kavitäten zueinander angeordnet sind, je mehr Auftragsvolumen für partikuläres Material steht je Umdrehungstakt der Dosierwalze für die Applikation zur Verfügung, oder je flacher können die Kavitäten ausgebildet werden, was sich im Hinblick auf eine vollständige Entleerung in der Abgabeposition als vorteilhaft erweisen kann.

## Patentansprüche

1. Verfahren zum Dosieren und Applizieren einer kleinen Menge eines partikulären Materials in einer schnelllaufenden Herstellungsmaschine (4) für absorbierende Hygieneartikel,
wobei in der Herstellungsmaschine (4) aufeinanderfolgend Komponenten (14) der herzustellenden Hygieneartikel als Zielstruktur (16) für das zu applizierende partikuläre Material zugeführt und gefördert werden,
wobei eine Dosierwalze (20) mit in ihrer Außenumfangsfläche (22) vorgesehenen Kavitäten (28) zur Aufnahme des partikulären Materials eingesetzt wird,
wobei die aufeinanderfolgend zugeführten Komponenten (14) an und vorzugsweise unterhalb der Dosierwalze (20) und vorzugsweise in einem Abstand zur Dosierwalze (20) vorbeigeführt werden,
**dadurch gekennzeichnet,**
**dass** die Dosierwalze (20) getaktet angesteuert wird, also entsprechend der Bewegung der vorbeigeführten Komponenten (14) beschleunigt und in jedem Takt bis zum Stillstand verzögert wird, so dass mit jedem Takt eine Anzahl von Kavitäten (28) in eine Abgabeposition (40) zu der gerade vorbeigeführten Komponente (14) gebracht und darin enthaltenes partikuläres Material an die Komponente (14) appliziert wird.

2. Verfahren nach Anspruch 1, **dadurch gekennzeichnet, dass** je nach Menge an zu applizierendem partikulären Material ein Drehwinkel der Dosierwalze (20) je Takt gewählt wird, wobei der Drehwinkel bevorzugt zwischen 5° und 30°, insbesondere zwischen 10° und 25° gewählt wird.

3. Verfahren nach einem oder mehreren der vorstehenden Ansprüche, **dadurch gekennzeichnet, dass** der Abgabeposition (40) der Kavitäten eine Leitanordnung (46) nachgeordnet ist, mittels derer aus den Kavitäten (28) ausgegebenes partikuläres Material in Richtung auf die Zielstruktur (16) oder einen Zielbereich der Zielstruktur (16) geleitet wird.

4. Verfahren nach Anspruch 3, **dadurch gekennzeichnet, dass** die Leitanordnung (46) eine Prall- oder Leitwand (48) aufweist, welche zu einer horizontalen Ebene einen Winkel zwischen 90° und 20°, insbesondere zwischen 90° und 25°, insbesondere zwischen 80° und 25°, insbesondere zwischen 60° und 25° und weiter insbesondere zwischen 50° und 25° einschließt.

5. Verfahren nach einem oder mehreren der vorstehenden Ansprüche, **dadurch gekennzeichnet, dass** sich nach innen verjüngende Kavitäten (28), insbesondere verrundet ausgebildete sich nach innen verjüngende Kavitäten (28), insbesondere kalottenförmig ausgebildete Kavitäten (28) bei der Dosierwalze vorgesehen werden.

6. Verfahren nach einem oder mehreren der vorstehenden Ansprüche, **dadurch gekennzeichnet, dass** die Kavitäten (28) in einem Übergang zu der Außenumfangsfläche (22) der Dosierwalze (20) von einer Wandung mit einem Öffnungswinkel von höchstens 60°, insbesondere von höchstens 50°, insbesondere von höchstens 45° und von wenigstens 25°, insbesondere von wenigstens 30°, insbesondere von wenigstens 35° begrenzt werden.

7. Verfahren nach einem oder mehreren der vorstehenden Ansprüche, **dadurch gekennzeichnet, dass** die Kavitäten (28) in der Außenumfangsfläche (22) der Dosierwalze (20) in parallel zu einer Antriebsachse (56) der Dosierwalze (20) verlaufenden Reihen (58) angeordnet sind.

8. Verfahren nach einem oder mehreren der vorstehenden Ansprüche, **dadurch gekennzeichnet, dass** die Kavitäten (28) in der Außenumfangsfläche (22) der Dosierwalze (20) in Gruppen (60) angeordnet sind, die in Umfangsrichtung voneinander beabstandet sind, so dass in Umfangsrichtung zwischen diesen Gruppen (60) ein kavitätenfreier Stegbereich (62) ausgebildet ist.

9. Vorrichtung (2) zum Dosieren und Applizieren einer kleinen Menge eines partikulären Materials in einer schnelllaufenden Herstellungsmaschine (4) für absorbierende Hygieneartikel,
wobei in der Herstellungsmaschine (4) aufeinanderfolgend Komponenten (14) der herzustellenden Hygieneartikel als Zielstruktur (16) für das zu applizierende partikuläre Material zugeführt und gefördert werden,
mit einer Dosierwalze (20) mit in ihrer Außenumfangsfläche (22) vorgesehenen Kavitäten (28) zur Aufnahme des partikulären Materials,
und mit einer Rakeleinrichtung (24) mit einer gegen die Außenumfangsfläche (22) der Dosierwalze (20) anliegenden Rakelschneide (26),
wobei die aufeinanderfolgend zugeführten Komponenten (14) an und vorzugsweise unterhalb der Dosierwalze (20) und vorzugsweise in einem Abstand zur Dosierwalze (20) vorbeigeführt werden, und mit einer Steuervorrichtung (50) und Antriebsvorrichtung (52) für die Dosierwalze (20),
**dadurch gekennzeichnet,**
**dass** die Steuervorrichtung (50) und Antriebsvorrichtung (52) derart ausgebildet ist, dass die Dosierwalze (20) getaktet ansteuerbar ist, also entsprechend der Bewegung der vorbeigeführten Komponenten (14) beschleunigt und in jedem Takt bis zum Stillstand verzögert wird, so dass mit jedem Takt eine Anzahl von Kavitäten (28) in eine Abgabeposition (40) zu der gerade vorbeigeführten Komponente gebracht und darin enthaltenes partikuläres Material an die Komponente (14) appliziert wird.

10. Vorrichtung nach Anspruch 9, **dadurch gekennzeichnet, dass** ein insbesondere vertikaler Abstand der an der Dosierwalze vorbeigeführten Komponenten und der Dosierwalze 1,0 - 15,0 cm, insbesondere 1,0 - 10,0, insbesondere 1,0 - 7,0 cm, insbesondere 1,0 - 5,0 cm, insbesondere 1,0 - 4,0 cm, insbesondere 1,0 - 3,0 cm, insbesondere 2,0 - 3,0 cm beträgt.

11. Vorrichtung nach Anspruch 9 oder 10, **dadurch gekennzeichnet, dass** der Abgabeposition (40) der Kavitäten (28) eine Leitanordnung (46) nachgeordnet ist, mittels derer aus den Kavitäten (28) ausgegebenes partikuläres Material in Richtung auf die Zielstruktur (16) oder einen Zielbereich der Zielstruktur (16) geleitet wird.

12. Vorrichtung nach Anspruch 11, **dadurch gekennzeichnet, dass** die Leitanordnung (46) eine Prall- oder Leitwand (48) aufweist, welche zu einer horizontalen Ebene einen Winkel zwischen 90° und 20°, insbesondere zwischen 90° und 25°, insbesondere zwischen 80° und 25°, insbesondere zwischen 60° und 25° und weiter insbesondere zwischen 50° und 25° einschließt.

13. Vorrichtung nach einem oder mehreren der Ansprüche 9-12, **dadurch gekennzeichnet, dass** bei der Dosierwalze (20) sich nach innen verjüngende Kavitäten (28), insbesondere verrundet ausgebildete sich nach innen verjüngende Kavitäten (28), insbesondere kalottenförmig ausgebildete Kavitäten (28) ausgebildet sind.

14. Vorrichtung nach einem oder mehreren der Ansprüche 9-13, **dadurch gekennzeichnet, dass** die Kavitäten (28) in einem Übergang zu der Außenumfangsfläche der Dosierwalze (20) von einer Wandung mit einem Öffnungswinkel von höchstens 60°, insbesondere von höchstens 50°, insbesondere von höchstens 45° und von wenigstens 25°, insbesondere von wenigstens 30°, insbesondere von wenigstens 35° begrenzt werden.

15. Vorrichtung nach einem oder mehreren der Ansprüche 9-14, **dadurch gekennzeichnet, dass** die Kavitäten (28) in der Außenumfangsfläche (22) der Dosierwalze (20) in parallel zu einer Antriebsachse (56) der Dosierwalze (20) verlaufenden Reihen (58) angeordnet sind.

16. Vorrichtung nach einem oder mehreren der Ansprüche 9-15, **dadurch gekennzeichnet, dass** die Kavitäten (28) in der Außenumfangsfläche (22) der Dosierwalze (20) in Gruppen (60) angeordnet sind, die in Umfangsrichtung voneinander beabstandet sind, so dass in Umfangsrichtung zwischen diesen Gruppen (60) ein kavitätenfreier Stegbereich (62) ausgebildet ist.

## Claims

1. Method for dosing and applying a small amount of a particulate material in a high-speed manufacturing machine (4) for absorbent hygiene articles,
in the manufacturing machine (4) components (14) of the hygiene articles to be produced being successively supplied and conveyed as target structure (16) for the particulate material to be applied,
a metering roller (20) which has cavities (28) provided in its outer circumferential surface (22) being used to receive the particulate material,
the successively supplied components (14) being guided past and preferably below the metering roller (20) and preferably at a distance from the metering roller (20), **characterized in that**
the metering roller (20) is controlled in a clocked manner, i.e. is accelerated in accordance with the movement of the components (14) being guided past and is decelerated to a standstill in each cycle, so that with each cycle a number of cavities (28) are brought into a dispensing position (40) to the component (14) just being guided past and particulate material contained therein is applied to the component (14).

2. Method according to claim 1, **characterized in that** depending on the quantity of particulate material to be applied, a rotation angle of the metering roller (20) is selected per cycle, the rotation angle being preferably selected between 5° and 30°, in particular between 10° and 25°.

3. Method according to one or more of the preceding claims, **characterized in that** a guide arrangement (46) is arranged downstream of the dispensing position (40) of the cavities, by means of which particulate material dispensed from the cavities (28) is guided in the direction of the target structure (16) or a target region of the target structure (16).

4. Method according to claim 3, **characterized in that** the guide arrangement (46) has a baffle or guide wall (48) which encloses an angle of between 90° and 20°, in particular between 90° and 25°, in particular between 80° and 25°, in particular between 60° and 25° and further in particular between 50° and 25°, with a horizontal plane.

5. Method according to one or more of the preceding claims, **characterized in that** inwardly tapering cavities (28), in particular rounded inwardly tapering cavities (28), in particular dome-shaped cavities (28) are provided in the metering roller.

6. Method according to one or more of the preceding claims, **characterized in that** the cavities (28) are bounded in a transition to the outer circumferential surface (22) of the metering roller (20) by a wall with an opening angle of at most 60°, in particular of at most 50°, in particular of at most 45° and of at least 25°, in particular of at least 30°, in particular of at least 35°.

7. Method according to one or more of the preceding claims, **characterized in that** the cavities (28) in the outer circumferential surface (22) of the metering roller (20) are arranged in rows (58) running parallel to a drive axis (56) of the metering roller (20).

8. Method according to one or more of the preceding claims, **characterized in that** the cavities (28) in the outer circumferential surface (22) of the metering roller (20) are arranged in groups (60) which are spaced apart from one another in the circumferential direction, so that a cavity-free web region (62) is formed between these groups (60) in the circumferential direction.

9. Device (2) for dosing and applying a small amount of a particulate material in a high-speed manufacturing machine (4) for absorbent hygiene articles,
in the manufacturing machine (4) components (14) of the hygiene articles to be produced being successively supplied and conveyed as target structure (16) for the particulate material to be applied,
comprising a metering roller (20) which has cavities (28) provided in its outer circumferential surface (22) for receiving the particulate material,
and comprising a doctor blade mechanism (24) with a doctor blade edge (26) resting against the outer circumferential surface (22) of the metering roller (20),
the successively supplied components (14) being guided past and preferably below the metering roller (20) and preferably at a distance from the metering roller (20), and comprising a control device (50) and drive device (52) for the metering roller (20),
**characterized in that**
the control device (50) and drive device (52) are designed such that the metering roller (20) can be controlled in a clocked manner, i.e. is accelerated in accordance with the movement of the components (14) being guided past and is decelerated to a standstill in each cycle, so that with each cycle a number of cavities (28) are brought into a dispensing position (40) to the component just being guided past and particulate material contained therein is applied to the component (14).

10. Device according to claim 9, **characterized in that** a particularly vertical distance between the components guided past the metering roller and the metering roller is 1.0 - 15.0 cm, in particular 1.0 - 10.0, in particular 1.0 - 7.0 cm, in particular 1.0 - 5.0 cm, in particular 1.0 - 4.0 cm, in particular 1.0 - 3.0 cm, in particular 2.0 - 3.0 cm.

11. Device according to claim 9 or 10, **characterized in that** a guide arrangement (46) is arranged downstream of the dispensing position (40) of the cavities (28), by means of which particulate material dispensed from the cavities (28) is guided in the direction of the target structure (16) or a target region of the target structure (16).

12. Device according to claim 11, **characterized in that** the guide arrangement (46) has a baffle or guide wall (48) which encloses an angle of between 90° and 20°, in particular between 90° and 25°, in particular between 80° and 25°, in particular between 60° and 25° and further in particular between 50° and 25°, with a horizontal plane.

13. Device according to one or more of claims 9-12, **characterized in that** in the metering roller (20) inwardly tapering cavities (28), in particular rounded inwardly tapering cavities (28), in particular dome-shaped cavities (28) are formed.

14. Device according to one or more of claims 9-13, **characterized in that** the cavities (28) are bounded in a transition to the outer circumferential surface of the metering roller (20) by a wall with an opening angle of at most 60°, in particular of at most 50°, in particular of at most 45° and of at least 25°, in particular of at least 30°, in particular of at least 35°.

15. Device according to one or more of claims 9-14, **characterized in that** the cavities (28) in the outer circumferential surface (22) of the metering roller (20) are arranged in rows (58) running parallel to a drive axis (56) of the metering roller (20).

16. Device according to one or more of claims 9-15, **characterized in that** the cavities (28) in the outer circumferential surface (22) of the metering roller (20) are arranged in groups (60) which are spaced apart from one another in the circumferential direction, so that a cavity-free web region (62) is formed between these groups (60) in the circumferential direction.

## Revendications

1. Procédé de dosage et d'application d'une petite quantité de matériau particulaire dans une machine de fabrication à grande vitesse (4) pour produits d'hygiène absorbants,
dans lequel des composants (14) des produits d'hygiène à fabriquer sont successivement amenés et acheminés dans la machine de fabrication (4) en tant que structure cible (16) pour le matériau particulaire à appliquer,
dans lequel on utilise un rouleau doseur (20) comprenant des cavités (28) prévues dans sa surface circonférentielle extérieure (22) pour recevoir le matériau particulaire,
dans lequel on fait passer les composants (14) amenés successivement, devant et de préférence en dessous du rouleau doseur (20), et de préférence à une distance du rouleau doseur (20),
**caractérisé par** le fait
le rouleau doseur (20) est commandé de manière cadencée, c'est-à-dire accélérée en fonction du mouvement des composants (14) qui passent devant, et est décéléré à chaque cadence jusqu'à l'arrêt de sorte que, à chaque cadence, un nombre de cavités (28) est amené dans une position de distribution (40) par rapport au composant (14) qui vient de passer, et que du matériau particulaire y contenu est appliqué sur le composant (14).

2. Procédé selon la revendication 1, **caractérisé par le fait que**, selon la quantité de matériau particulaire à appliquer, un angle de rotation du rouleau doseur (20) est sélectionné par cadence, dans lequel l'angle de rotation est choisi de préférence entre 5° et 30°, en particulier entre 10° et 25°.

3. Procédé selon une ou plusieurs des revendications précédentes, **caractérisé par le fait qu'**un dispositif de guidage (46) est disposé en aval de la position de distribution (40) des cavités, au moyen duquel du matériau particulaire distribué par les cavités (28) est guidé en direction de la structure cible (16) ou une zone cible de la structure cible (16).

4. Procédé selon la revendication 3, **caractérisé par le fait que** le dispositif de guidage (46) comprend une paroi chicane ou de guidage (48) qui forme un angle compris entre 90° et 20°, en particulier entre 90° et 25°, en particulier entre 80° et 25°, en particulier entre 60° et 25°, et en outre en particulier entre 50° et 25°, avec un plan horizontal.

5. Procédé selon une ou plusieurs des revendications précédentes, **caractérisé par le fait que** des cavités (28) se rétrécissant vers l'intérieur, en particulier des cavités (28) se rétrécissant vers l'intérieur et réalisées de manière arrondie, en particulier des cavités (28) réalisées en forme de calotte, sont prévues dans le rouleau doseur.

6. Procédé selon une ou plusieurs des revendications précédentes, **caractérisé par le fait que** les cavités (28) sont délimitées, dans une transition vers la surface circonférentielle extérieure (22) du rouleau doseur (20), par une paroi présentant un angle d'ouverture de 60° tout au plus, en particulier de 50° tout au plus, en particulier de 45° tout au plus, et d'au moins 25°, en particulier d'au moins 30°, en particulier d'au moins 35°.

7. Procédé selon une ou plusieurs des revendications précédentes, **caractérisé par le fait que** les cavités (28) sont disposées en rangées (58) dans la surface circonférentielle extérieure (22) du rouleau doseur (20), qui s'étendent parallèlement à un axe d'entraînement (56) du rouleau doseur (20).

8. Procédé selon une ou plusieurs des revendications précédentes, **caractérisé par le fait que** les cavités (28) sont disposées en groupes (60) dans la surface circonférentielle extérieure (22) du rouleau doseur (20), qui sont espacés les uns des autres dans le sens circonférentiel de manière à former une zone d'entretoise (62) exempte de cavités entre ces groupes (60) dans le sens circonférentiel.

9. Dispositif (2) de dosage et d'application d'une petite quantité de matériau particulaire dans une machine de fabrication à grande vitesse (4) pour produits d'hygiène absorbants,
dans lequel des composants (14) des produits d'hygiène à fabriquer sont successivement amenés et acheminés dans la machine de fabrication (4) en tant que la structure cible (16) pour le matériau particulaire à appliquer,
avec un rouleau doseur (20) comprenant des cavités (28) prévues dans sa surface circonférentielle extérieure (22), pour recevoir le matériau particulaire,
et avec un dispositif de racle (24) comprenant un tranchant de racle (26) qui s'appuie contre la surface circonférentielle extérieure (22) du rouleau doseur (20),
dans lequel on fait passer les composants (14) amenés successivement, devant et de préférence en dessous du rouleau doseur (20), et de préférence à une distance du rouleau doseur (20), et avec un dispositif de commande (50) et un dispositif d'entraînement (52) pour le rouleau doseur (20),
**caractérisé par le fait**
**que** le dispositif de commande (50) et le dispositif d'entraînement (52) sont conçus de telle manière que le rouleau doseur (20) peut être commandé de manière cadencée, c'est-à-dire accélérée en fonction du mouvement des composants (14) qui passent devant, et est décéléré à chaque cadence jusqu'à l'arrêt de sorte que, à chaque cadence, un nombre de cavités (28) est amené dans une position de distribution (40) par rapport au composant (14) qui vient de passer, et que du matériau particulaire y contenu est appliqué sur le composant (14).

10. Dispositif selon la revendication 9, **caractérisé par le fait qu'**une distance en particulier verticale entre les composants que l'on fait passer devant le rouleau doseur et le rouleau doseur est comprise entre 1,0 et 15,0 cm, en particulier entre 1,0 et 10,0 cm, en particulier entre 1,0 et 7,0 cm, en particulier entre 1,0 et 5,0 cm, en particulier entre 1,0 et 4,0 cm, en particulier entre 1,0 et 3,0 cm, en particulier entre 2,0 et 3,0 cm.

11. Dispositif selon la revendication 9 ou 10, **caractérisé par le fait qu'**un dispositif de guidage (46) est disposé en aval de la position de distribution (40) des cavités (28), au moyen duquel du matériau particulaire distribué par les cavités (28) est guidé en direction de la structure cible (16) ou une zone cible de la structure cible (16).

12. Dispositif selon la revendication 11, **caractérisé par le fait que** le dispositif de guidage (46) comprend une paroi chicane ou de guidage (48) qui forme un angle compris entre 90° et 20°, en particulier entre 90° et 25°, en particulier entre 80° et 25°, en particulier entre 60° et 25°, et en outre en particulier entre 50° et 25°, avec un plan horizontal.

13. Dispositif selon une ou plusieurs des revendications 9 à 12, **caractérisé par le fait que** des cavités (28) se rétrécissant vers l'intérieur, en particulier des cavités (28) se rétrécissant vers l'intérieur et réalisées de manière arrondie, en particulier des cavités (28) réalisées en forme de calotte, sont réalisées dans le rouleau doseur (20).

14. Dispositif selon une ou plusieurs des revendications 9 à 13, **caractérisé par le fait que** les cavités (28) sont délimitées, dans une transition vers la surface circonférentielle extérieure du rouleau doseur (20), par une paroi présentant un angle d'ouverture de 60° tout au plus, en particulier de 50° tout au plus, en particulier de 45° tout au plus, et d'au moins 25°, en particulier d'au moins 30°, en particulier d'au moins 35°.

15. Dispositif selon une ou plusieurs des revendications 9 à 14, **caractérisé par le fait que** les cavités (28) sont disposées en rangées (58) dans la surface circonférentielle extérieure (22) du rouleau doseur (20), qui s'étendent parallèlement à un axe d'entraînement (56) du rouleau doseur (20).

16. Dispositif selon une ou plusieurs des revendications 9 à 15, **caractérisé par le fait que** les cavités (28) sont disposées en groupes (60) dans la surface circonférentielle extérieure (22) du rouleau doseur (20), qui sont espacés les uns des autres dans le sens circonférentiel de manière à former une zone d'entretoise (62) exempte de cavités entre ces groupes (60) dans le sens circonférentiel.
